(19) Europäisches Patentamt
European Patent Office
Office européen des brevets

(11) **EP 2 505 126 A1**

(12) **EUROPEAN PATENT APPLICATION**

(43) Date of publication:
03.10.2012 Bulletin 2012/40

(51) Int Cl.:
*A61B 3/032* (2006.01)   *A61B 3/08* (2006.01)
*A61B 3/11* (2006.01)   *H04N 13/00* (2006.01)

(21) Application number: 12162255.9

(22) Date of filing: 29.03.2012

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(30) Priority: 31.03.2011 JP 2011081288

(71) Applicant: NIDEK CO., LTD
Gamagori-shi
Aichi 443-0038 (JP)

(72) Inventor: **Hirayama, Yukito**
Okazaki-shi
Aichi, 444-0826 (JP)

(74) Representative: **Zimmermann, Tankred Klaus et al**
Schoppe, Zimmermann, Stöckeler
Zinkler & Partner
P.O. Box 246
82043 Pullach (DE)

(54) **Optotype presenting apparatus**

(57)   An optotype presenting apparatus presents optotypes for testing an examinee's binocular visual function. The apparatus includes: an optotype presenting part to display an optotype at a predetermined presenting region; a pupillary distance input part that inputs a pupillary distance between an examinee's left and right eyes; and a control unit that makes the optotype presenting part display left- and right-eye optotypes for a stereoscopic vision test. The control unit determines a lateral inter-optotype spacing between the left- and the right-eye optotypes based on the input pupillary distance so that a fusion optotype seen with the examinee's both eyes is perceived as an optotype that is seen floating or sinking by a fusion distance between a reference position and a predetermined fusion position, and makes the optotype presenting part display the left-eye optotype and the right-eye optotype based on the determined inter-optotype spacing.

FIG. 6

**Description**

CROSS-REFERENCE TO RELATED APPLICATION

This application is based on Japanese Patent Application No. 2011-081288 filed with the Japan Patent Office on March 31, 2011, the entire content of which is hereby incorporated by reference.

BACKGROUND

1. Technical Field

The present disclosure relates to an optotype presenting apparatus to present optotypes to be used for a visual function test to an examinee.

2. Related Art

In recent years, an optotype presenting apparatus has been developed, which presents (displays) optotypes on a display, such as a liquid crystal panel, to test the visual functions, such as visual acuity, of an examinee (see, for example, JP-A-2009-207569 (EP 2095760),). Such an apparatus is able to present optotypes of various kinds, shapes, sizes, and so on to the examinee's left and right eyes by changing optotypes displayed on the display,. Furthermore, the apparatus separates optotypes into a left-eye optotype to be presented to examinee's left eye and a right-eye optotype to be presented to examinee's right eye by means of polarization or the like. By displaying the left-eye optotype and the right-eye optotype on the display at a predetermined display spacing (inter-optotype spacing), a parallax is produced in examinee's left and right eyes. As a result, the examinee perceives the flotage (or the sinkage) of the optotype on the display. In this way, a binocular visual function test, such as a stereoscopic visual function test, can be performed.

SUMMARY

An optotype presenting apparatus that presents optotypes for testing an examinee's binocular visual function includes: an optotype presenting part to display an optotype at a predetermined presenting region; a pupillary distance input part that inputs a pupillary distance between an examinee's left and right eyes; and a control unit that makes the optotype presenting part display a left-eye optotype and a right-eye optotype for a stereoscopic vision test, wherein the control unit determines a lateral inter-optotype spacing between the left-eye optotype and the right-eye optotype based on the input pupillary distance so that a fusion optotype seen with the examinee's both eyes is perceived as an optotype that is seen floating or sinking by a fusion distance between a reference position and a predetermined fusion position, and makes the optotype presenting part display the left-eye optotype and the right-eye optotype based on the determined inter-optotype spacing.

BRIEF DESCRIPTION OF THE DRAWINGS

Fig 1 is a schematic external view of an optotype presenting apparatus according to an embodiment;
Fig. 2 is a block diagram schematically illustrating the configuration of an optotype presenting part and the configuration of a control system of the optotype presenting apparatus;
Fig. 3 is a schematic front view of an optotype as a trirod vision-testing optotype displayed at the optotype presenting apparatus;
Fig. 4 is a schematic diagram illustrating the relationship between the trirod vision-testing optotype displayed at the optotype presenting apparatus and a pupillary distance of an examinee;
Fig. 5 is a schematic diagram illustrating subpixel-processing performed at the optotype presenting apparatus; and
Fig. 6 is a schematic perspective view of a state in which the trirod vision-testing optotype is seen with both the eyes of an examinee.

DESCRIPTION OF EMBODIMENTS

In the following detailed description, for purpose of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

When optotypes that produce a parallax such as that described earlier have been presented to the right and left eyes of an examinee, the examinee perceives the flotage (or the sinkage) of the optotypes. In the case where examinees differ in pupillary distance, however, the examinees perceive the flotage amounts (distances) or the sinkage amounts (distances) of optotypes differently from one another. That is, the fusion position of optotypes that produce a parallax is differently perceived by the right and left eyes of the respective examinees having different pupillary distances.

An object of the present invention is to provide an optotype presenting apparatus capable of performing a more accurate visual acuity test by optimally adjusting the fusion position of optotypes that produce a parallax based on the pupillary distance of an examinee.

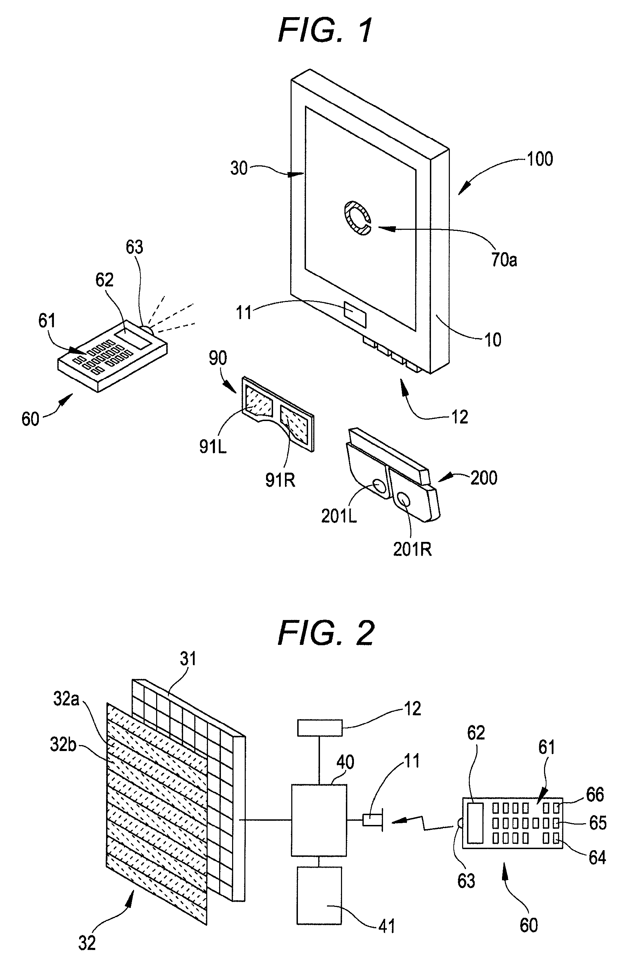

An optotype presenting apparatus according to an embodiment of the present invention will be described below with reference to the accompanying drawings. Fig. 1 is a schematic external view of the optotype presenting apparatus 100. On the front of a housing 10 of the optotype presenting apparatus 100, an optotype presenting part (optotype presenting means or an optotype presenting unit) 30 is provided. The optotype presenting part 30 has a screen (a predefined presenting region). Specifically, the screen is the screen of a display 31 placed within the optotype presenting part 30 as a presenting unit (display means) (see Fig. 2). Incidentally, for example, the display 31 used may be a 19- or 17-inch color liquid crystal display having a resolution of SXGA. In addition, the housing 10 can be shaped thinly so as to be hanged on a wall. The screen of the display 31 has a sufficient size even when placed at a distant position, for example at a position 6 meters away from the examinee, to enable the presentation (the display) of optotypes such as a predetermined-size optotype 70a for visual acuity testing (e.g., Landolt's ring prototype representing a VA (visual acuity) of 2.0).

At a lower portion of the front of the housing 10, a receiving part 11 is provided as a receiving unit (receiving means). The receiving part 11 receives an infrared communication signal from a remote controller (hereinafter referred to as "remote control") 60 served as an operating unit (operating means or an operating part). Furthermore, at the bottom of the housing 10, function keys (buttons) 12 are provided. The function keys 12 are used as a setting (input) unit (a setting (input) part or setting (input) means) to carry out various settings on the apparatus 100 (input of various items to the apparatus 100). By using one of the function keys 12, for example, an installation distance (the distance from an examinee (the right and left eyes of an examinee) to the optotype presenting part 30) can be set (input).

The optotype presenting apparatus 100 is provided with the remote control 60. The remote control 60 includes a plurality of keys (buttons) 61, a liquid crystal display 62, and a transmitting part 63. The plurality of keys 61 is used as a selecting unit (optotype selecting means or an optotype selecting part (selector)) to select an optotype to be presented (displayed) on the optotype presenting part 30 (the display 31). The liquid crystal display 62 is a display unit (a display part or display means) that displays information such as a selected optotype. The transmitting part 63 sends infrared light (an optical communication signal). The remote control 60 further includes an installation distance input key (button) 64, a pupillary distance input key (button) 65, and a fusion distance input key (button) 66. The installation distance input key (button) 64 is an input unit (an input part or input means) to input an installation distance set for the apparatus 100. The pupillary distance input key (button) 65 is an input unit (an input part or input means) to input a (pupillary distance (PD) of an examinee. The fusion distance input key (button) 66 is an input unit (an input part or input means) to input the fusion distance between optotypes that produce a parallax. Incidentally, optotypes for stereoscopic vision test to be presented to the examinee include a left-eye optotype to be presented to the left eye of the examinee and a right-eye optotype to be presented to the right eye of the examinee. At the time of a stereoscopic vision test, the left-eye optotype and the right-eye optotype are presented (displayed) on the optotype presenting part 30 (the display 31) with a predetermined distance (inter-optotype distance) kept laterally between the two optotypes. The examinees perceive the left-eye optotype with the left eye, and perceive the right-eye optotype with the right eye. The examinee fuses the two optotypes presented to the left and right eyes together (see both the optotypes as one image), and therefore perceive the flotage (or the sinkage) of the optotypes. In addition, the term "fusion distance" refers to the distance from a reference plane (a reference position, or the display surface of the display 31 according to this embodiment) to an optotype that is seen floating or to an optotype that is seen sinking. Alternatively, the fusion distance to be used may be the distance from an examinee to an optotype that is seen floating or an optotype that is seen sinking.

Fig. 2 is a schematic block diagram illustrating the configuration of the optotype presenting part 30 and the configuration of the control system of the optotype presenting apparatus 100. The optotype presenting part 30 of Fig. 2 includes the display (the color liquid crystal display) 31 and a polarization optical member 32. The display 31 includes a plurality of pixels. Each pixel includes laterally (horizontally) arranged subpixels that respectively emit red light, blue light, and green light (which emit light of the three primary colors). Thus, a single pixel can display various colors. The polarization optical member 32 is a sheet-shaped member placed on (adhered to) the front side of the display 31, and has a sufficient size to cover at least the entire optotype representing (display) region of the display 31.

The control unit (the controlling part or the controlling means) 40 of the apparatus 100 is connected to the display 31, the receiving part 11, and the function keys 12. The control unit 40 also connects to a memory 41, a decoder circuit (not shown), and so on. The memory 41 is a storage unit (a storage part or storage means) that sores the data of various optotypes. The decoder circuit (not shown) decodes command signals from the remote control 60. The control unit 40 controls the representation of pixels on the display 31 based on optotype-switching command signals and so on input from the remote control 60. Furthermore, the control unit 40 performs subpixel-processing on the individual pixels at the boundary portion of an optotype (the boundary between the optotype and the background) displayed on the display 31. As a result, the examinee sees (perceives) the optotype as if the display position (the presentation position) of the optotype is changed (details of such a thing will be described later).

The configuration of the polarization optical member 32 will be described below. The display 31 emits linearly polarized light. Linearly polarized light has a polarizing axis in a predetermined direction (the vertical direction, the horizontal direction, or a 45-degree oblique direction). In this embodiment, linearly polarized light having a vertical polarizing axis is emitted. The polarization optical member 32 has line-shaped optical regions 32a and line-shaped optical regions 32b. The optical regions 32a and 32b each extend laterally so as to correspond to predetermined times the size of a single pixel on the display 31. Furthermore, the optical regions 32a and the optical regions 32b are arranged longitudinally (vertically) and alternately. Light emitted from the display 31 passes through the optical regions 32a and the optical regions 32b. At that time, the optical regions 32a and the optical regions 32b convert the light to linearly polarized light. The polarizing axis of the linearly polarized light obtained by passing the light through the optical regions 32a and the polarizing axis of the linearly polarized light obtained by passing the light through the optical regions 32b are perpendicular to each other. The polarization optical member 32 has the same phase difference-causing effect as that of a half-wave plate. As well known in the art, a half-wave plate turns the vibration direction of incident light by $2 \times \theta$ degrees. The letter "θ" used herein refers to the angle formed by the polarizing axis of incident light and the fast axis (the slow axis) of the half-wave plate. That is, the half-wave plate has an optically principal axis as a fast axis or a slow axis. The optically principal axis of the half-wave plate is inclined to the direction of the polarizing axis of incident light. That is, half-wave plates have characteristics of being able to turn the direction of the polarizing axis (the direction of the oscillation) of incident light by a predetermined angle with respect to their optically principal axis while keeping the amount of the incident light constant.

An examinee wears polarizing spectacles 90 at the time of a binocular visual function test (e.g., a stereoscopic vision test). The polarizing spectacles 90 are provided with a polarizing filter 91L and a polarizing filter 91R: the polarizing axis of the polarizing filter 9 1 L is perpendicular to the polarizing axis of the polarizing filter 91 R. The polarizing filter 91 L is placed in front of the left eye of the examinee, and the polarizing filter 91R is placed in front of the right eye of the examinee. In this embodiment, the direction of the polarizing axis of the polarizing filter 91L is 45 degrees, and the direction of the polarizing axis of the polarizing filter 91R is 135 degrees. Furthermore, instead of the polarizing spectacles 90, a subjective refractivity measuring device (hereinafter referred to as "phoropter") 200 may be used. The phoropter 200 has a left test window and a right test window. When using the phoropter 200, the left test window is set in front of the left eye of the examinee, and the right test window is set in front of the right eye of the examinee. In the phoropter 200, switching between spherical lenses cylindrical lenses, auxiliary lenses, and so on is performed at the left and right test windows. More specifically, the phoropter 200 is provided with a polarizing filter 201 L (having a polarizing axis in a 45-degree direction) and a polarizing filter 201 R (having a polarizing axis in a 135-degree), and both the polarizing axes are perpendicular to each other. The polarizing filter 201L is placed at the left test window of the phoropter 200, while the polarizing filter 20 1 R is placed at the right test window.

The optical regions 32a of the polarization optical member 32 are left-eye optical regions. In this embodiment, the direction of the optically principal axes of the optical regions 32a is set at a predetermined angle. Therefore the direction of the polarizing axis of light incident from the display 31 is made to correspond with the direction of the polarizing axis of the left-eye polarizing filter 9 1 L of the polarizing spectacles 90 (or the direction of the polarizing axis of the left-eye polarizing filter 201 L of the phoropter 200) (the direction of 45 degrees). On the other hand, the optical regions 32b are right-eye optical regions. In this embodiment, the direction of the optically principal axes of the optical regions 32b is also set at a predetermined angle. Thus the direction of the polarizing axis of light incident from the display 31 is made to correspond with the direction of the polarizing axis of the right-eye polarizing filter 91R of the polarizing spectacles 90 (or the direction of the polarizing axis of the right-eye polarizing filter 201R of the phoropter 200) (the 135-degree direction). Therefore, when the examinee has looked at the optotype presenting part 30 through the polarizing filters 9 1 L and 9 1 R (or the polarizing filters 201 L and 20 1 R) set in front of the left and right eyes, light from the optical regions 32a having passed through the polarizing filter 91L (or the polarizing filter 201L) is perceived by the left eye. In contrast, light from the optical regions 32b is intercepted by the polarizing filter 91 L or 201 L and is, hence, not perceived by the left eye: the light from the optical regions 32b having passed through the polarizing filter 9 1 R (or the polarizing filter 20 1 R) is perceived by the right eyes. In contrast, the light from the optical regions 32a is intercepted by the polarizing filter 91R or 201R and is, hence, not perceived by the right eye. Since the light from the display 31 is separated and then enters the left and right eyes of the examinee in this way, different optotypes are respectively presented to the left and right of the examinee; that is, optotypes that produce a parallax are presented to the left and right eyes of the examinee.

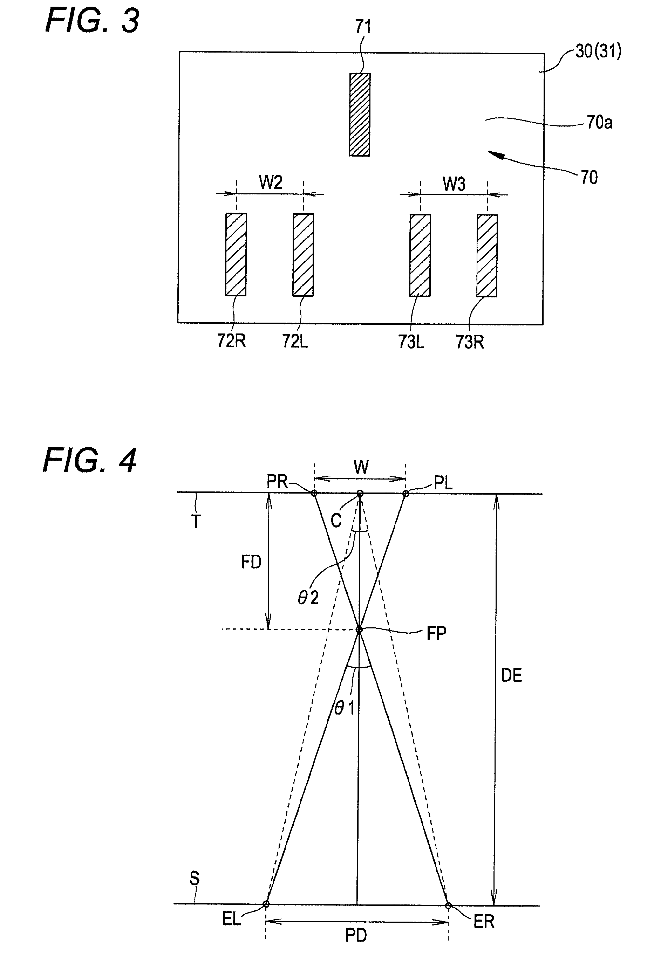

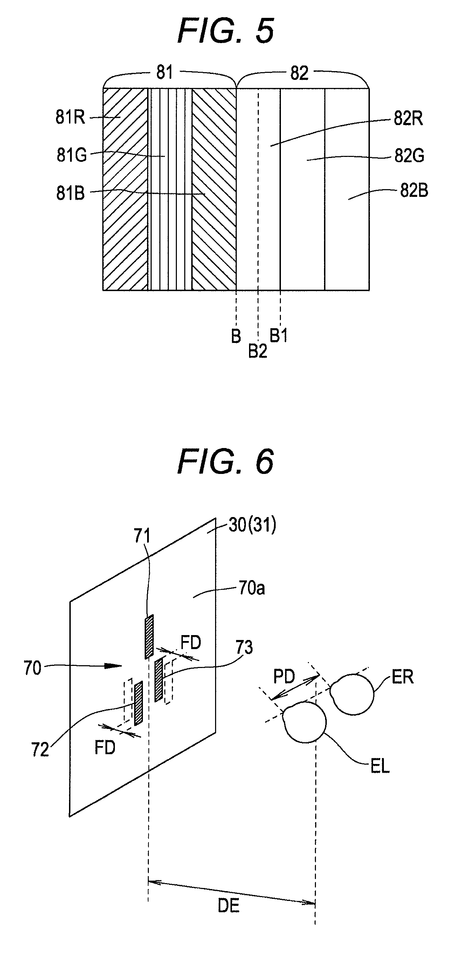

Next, a trirod vision-testing optotype presented (displayed) on the display 31 will be described below. Fig. 3 is a schematic view of the trirod vision-testing optotype (image) according to this embodiment. Fig. 4 is a schematic diagram illustrating the relationship between the trirod vision-testing optotype displayed at the optotype presenting apparatus 100 and a pupillary distance of an examinee. Fig. 5 is an explanatory drawing of subpixel-processing. Fig. 6 is a schematic diagram illustrating a state in which the trirod vision-testing optotype is seen with both the eyes of the examinee.

In this embodiment, the test distance (the distance from examinees to the apparatus 100) DE is 2.5 meters. The flotage amount or the sinkage amount of the optotype used as a test reference is 2 centimeters. Furthermore, the installation distance for the apparatus 100 can be set to 2.5 to 6.0 meters in 50-centimeter steps.

The optotype (the image) 70 according to this embodiment is constituted by three types of optotypes to conduct a simplified trirod vision test: the optotypes are shaped like a rod (bar) so that such a test designed to resemble a trirod vision test using a trirod vision tester can be conducted. Specifically, the optotype 70 is constituted by an optotype (first optotype) 71, an optotype (second optotype) 72, and an optotype (third optotype) 73. The optotype (first optotype) 71 can be seen (is perceived) at a first position a distance from the examinee (at a test distance DE). The optotype (first optotype) 71 is used as a test reference and, therefore, also referred to as "reference optotype 71". The optotype (second optotype) 72 is seen (perceived) by the examinee as if the optotype 72 floated at a predetermined distance (a fusion distance FD) from the reference optotype 71. The optotype (third optotype) 73 is seen (perceived) by the examinee as if the optotype 73 sank at the fusion distance FD from the reference optotype 71. A background 70a for the optotype 70 has a white color. All the optotypes 71, 72, and 73 are identical in color (have a black color; moreover, the optotypes 71, 72, and 73 are identical in shape and size.

The optotype 71 is a single optotype, and is displayed at a position where the position of the optotype 71 perceived by the left eye of the examinee coincides with the position of the optotype 71 perceived by the right eye. The optotype 72 is composed of a left-eye optotype 72L and a right-eye optotype 72R. The optotype 72L and the optotype 72R are spaced from each other by a predetermined spacing W2. The optotypes 72L and 72R are identical in shape, size, and color so that the optotype 72 can be seen with both the eyes of the examinee as one fusion optotype. The optotype 73 is composed of a left-eye optotype 73L and a right-eye optotype 73R. The optotype 73L and the optotype 73R are spaced from each other by a predetermined spacing W3. The optotypes 73L and 73R are identical in shape, size, and color so that the optotype 73 can be seen with both the eyes of the examinee as one fusion optotype. The spacing W2 is determined based on the test distance DE and the pupillary distance PD of an examinee. That is, when providing the spacing W2, the optotype 72 is seen (perceived) floating from the screen of the optotype representing part 30 by a fixed distance (the fusion distance FD) even in the case where an examinee has any pupillary distance PD. Likewise, the spacing W3 is determined based on the test distance DE and the pupillary distance PD of an examinee. That is, when providing the spacing W3, the optotype 73 is seen (perceived) sinking from the screen of the optotype representing part 30 by the fixed distance (the fusion distance FD) even in the case where an examinee has any pupillary distance PD.

By using the optotype presenting apparatus 100 according to this embodiment, a vision test, which is designed to resemble a trirod vision test using a trirod vision tester, can be conducted. That is, the test distance DE for the optotype presenting apparatus 100 is set to 2.5 meters. Furthermore, both the flotage amount of the optotype 72 and the sinkage amount of the optotype 73 are set to 2 centimeters regardless of the pupillary distance PD of an examinee. The test distance DE is equal to the distance from the eyes of examinees to left and right rods fixedly displayed at the time of trirod vision tests using a trirod vision tester. The fusion distance FD (the flotage amount and the sinkage amount) for the optotype presenting apparatus 100 is set based on a condition under which it is determined that the eyes of examinee have deep visual acuity at the time of trirod vision test using a trirod vision tester. That is, in trirod vision test using a trirod vision tester, having good vision or having bad vision is determined based on whether an examinee can stop the movement of a central rod at a position within 2 centimeters in front of and behind left and right rods with both eyes. Because of this, the fusion distance FD for the optotype presenting apparatus 100 is set in such a way that such a condition is met.

In this embodiment, the optotype 70 is stored in the memory 41 as vector data. The control unit 40 calculates (get) the inter-optotype spacings (the inter-optotype distances) W2 and W3 based on the test distance DE, the pupillary distance PD, and the fusion distance FD. Then the control unit 40 performs control so that the optotype 70 is formed based on the calculated inter-optotype spacings W2 and W3, and then presented at the optotype presenting part 30 (displayed on the display 31).

Nest, the relationship between the pupillary distance PD and the fusion distance FD will be described below. In the following, a case where an examinee perceives the flotage of the optotype will be described. Plural points in Fig. 4 indicate various positions. Reference letter S denotes a plane on which a left eye EL and a right eye ER are aligned. Reference letter T denotes a plane on which a left-eye optotype PL and a right-eye PR are aligned (the plane means the screen of the optotype presenting part 30). The plane S and the plane T are opposite to each other at a fixed distance (the test distance DE). The fusion position of the optotype is represented as the intersection point of the line segment joining the left eye EL and the optotype PL and the line segment joining the right eye ER and the optotype PR. The fusion distance FD refers to the distance between the plane T and the fusion position FP. The pupillary distance PD refers to the spacing between the pupil of the left eye EL and the pupil of the right eye ER. The inter-optotype spacing W refers to the spacing between the center of the optotype PL and the center of the optotype PR.

A parallax produced at the time of a stereoscopic vision test will be described below. An angle θ1 in Fig. 4 is the angle formed by the line segment joining the left eye EL and the fusion position FP and the line segment joining the right eye ER and the fusion position FP. An angle θ2 is the angle formed by the line segment joining the left eye EL and the center C of the plane T and the line segment joining the right eye ER and the center C. Therefore the parallax refers to the difference between the angle θ1 and the angle θ2. Hence, in the case where the test distance DE and the inter-optotype W are fixed, the parallax is fixed regardless of the pupillary distances PD, but the fusion distance FD changes with the pupillary distance PD. That is, the relationship between the pupillary distance PD and the fusion distance FD is represented by Equation 1 below based on two triangles shown in Fig. 4.

$$\text{PD} : \text{W} = (\text{DE} - \text{FD}) : \text{FD} \qquad \text{[Equation 1]}$$

where "DE - FD" represents a distance between an examinee and the fusion position FP. Expression 2 below is obtained by solving Equation 1 for the fusion distance FD.

$$\text{FD} = \text{W·DE/(PD + W)} \qquad \text{[Equation 2]}$$

As is evident from Expression 2, the fusion distance FD depends on the pupillary distance PD and the inter-optotype spacing W. Expression 3 below is obtained by solving Equation 1 for the inter-optotype spacing W.

$$\text{W} = \text{PD·FD/(DE} - \text{FD)} \qquad \text{[Equation 3]}$$

In this embodiment, the fusion distance FD is fixed (2 centimeters), and the pupillary distance PD is a variable. The control unit 40 determines an inter-optotype spacing W that fix the fusion distance FD, and then forms an optotype under such a condition.

Next, an exemplary method for adjusting the inter-optotype spacing W will be described below. For example, in the following will be described the difference in inter-optotype spacing W between the case where the pupillary distance PD is 60 millimeters and the case where the pupillary distance PD is 59 millimeters under conditions that the fusion distance FD is fixed (2 centimeters) and the pupillary distance PD is input in 1-millimeter steps. In the case where FD = 20 mm and DE = 2500 mm in Equation 3, W = 0.484 mm when PD = 60 mm; on the other hand, when PD = 59 mm, W = 0.476 mm. That is, the pupillary distance PD and the inter-optotype spacing W has a linear relationship and therefore, in the case where the pupillary distance PD has been changed by 1 millimeter, the inter-optotype spacing W can be changed by about 0.008 millimeters. Hence, where the test distance DE is 2.5 meters, the optotype presenting part 30 (the display 31) can have a resolution of 0.008 millimeters. The optotype presenting part 30 (the display 31) can present an optotype corresponding to the pupillary distance PD that is changed in 1-millimeter steps.

A method for adjusting and displaying the inter-optotype spacing W will be described below. Fig. 5 is a schematic diagram illustrating subpixel-processing performed at the optotype presenting apparatus 100. In Fig. 5 are schematically shown two pixels provided at the boundary (i.e., the lateral boundary portion) between the optotype 72 or 73 and the background in the case where the optotype 72 or 73 is presented to any one of the left and right eyes. As shown in Fig. 5, the pixels 81 and 82 are each composed of three subpixels. The three subpixels are arranged laterally, and differ in color. That is, the subpixels 81R and 82R display a red color (emit red light), the subpixels 81G and 82G display a green color (emit green light), and the subpixels 81B and 82B display a blue color (emit blue light). The subpixels can be made to individually display the colors (emit light) at 256 levels of brightness. That is, the brightness of the subpixels can be represented in the form of a level of 0 (zero) (that means black color) to a level of 255 (that means white color). When the brightness levels of all the subpixels are the level of 0 (zero), the examinee sees (perceives) the pixels to be black. When the brightness levels of all the subpixels are the level of 255, the examinee sees (perceives) the pixels to be white.

As shown in Fig. 5, there is a boundary portion B between the pixel 81 and the pixel 82. With the pixel 81, the brightness levels of all the subpixels (81R, 81G, and 81B) are the level of 0 (zero); thus the examinee sees (perceives) the pixel 81 to be black. On the other hand, the brightness levels of all the subpixels (82R, 82G, and 82B) of the pixel 82 are the level of 255; thus the examinee sees (perceives) the pixel 82 to be white. In that case, the examinee perceives the outline of the optotype 72 or 73 on the boundary portion B.

In the above state, the subpixel 82R of the pixel 82 adjoins the pixel 81. When the brightness of the subpixel 82R has been set at the level of 0 (zero), the examinees sees (perceives) the pixel 82 to be cyan; however, the examinee sees (perceives) the subpixel 82R to be black. Therefore the examinee sees (perceives) the position of the outline of the optotype 72 or 73 as if the position changed to a boundary portion B 1 between the subpixel 82R and the subpixel 82G beyond the boundary portion B. At that time, it is difficult for the examinee to clearly see (perceive) the pixel 82 to be cyan. This is because the optotype 72 or 73 is displayed (seen) in black color, the periphery of the optotype 72 or 73 is displayed (seen) in white color, and the pixel 82 is nothing but one of the pixels. Likewise, the examinee perceives a change in the position of the outline of the optotype 72 or 73 when the brightness of the subpixel 82G or 82B has been set at the level of 0 (zero),

By changing the brightness of the subpixels of one pixel like this, the position of the outline of the optotype 72 or 73 (the inter-optotype spacing) can be changed in the order of the number of the subpixels of one pixel. When a width that is an integral multiple of the width of the pixel of the display 31 is small compared with the inter-optotype spacing, the control unit 40 performs subpixel-processing on at least one of the left-eye optotype and the right-eye optotype. For example, when the control unit 40 performs subpixel-processing on the perimeter (the longitudinal portions of the perimeter) of one of the above two optotypes, the subpixel-processing is performed on the longitudinal portions of the outline (the inner portions of both the long sides) of the optotype. The control unit 40 calculates the brightness of the pixels (the brightness of the subpixels) at the perimeter of the optotype, and controls the display of the optotype on the display 30. Therefore the stereoscopic vision testing optotype can be presented to the examinee at the inter-optotype spacing (corresponding to the pupillary distance PD of the examinee) calculated by the control unit 40.

In the above description, the brightness of the subpixels has been set at the level of 0 or 255; and furthermore, by changing the brightness of the subpixels in stages too, the position of the outline of the optotype 72 or 73 can be changed. For example, the 256 levels of brightness of the subpixels are equally divided into 8 steps; that is, there are 32 levels of brightness for each step. For example, when t the subpixel 82R has a brightness level of 127, the amount of light emission at the portion between the boundary portion B and the boundary portion B1 becomes nearly half. As a result, between the subpixel 82R and the subpixel 82G, a difference in amount of light emission (shade of color) is made. Therefore the examinee sees (perceives) the optotype 72 or 73 as if the outline of the optotype 72 or 73 is positioned at a boundary portion B2 between the boundary portion B and the boundary portion B 1. That is, the examinee can perceive the position of the outline of the optotype 72 changed to the portion from the boundary portion B to the boundary portion B 1 in accordance with the brightness of the subpixel 82R (the difference in shade of color between the subpixels). Such a perception is also effected at the subpixels 82G and 82B. And further, the brightness levels of the subpixels of the subpixel may be changed from the side of the optotype to the side of the background in order (in the order of from 82R to 82B). In that case, the position of the boundary (the outline) changes from the side of the optotype to the side of the background. That is, the examinee perceives the optotype as if the position of the boundary (the outline) changed from the side of the optotype to the side of the background.

The examinee can be made to perceive the position of the outline of the optotype changed within one subpixel in this way. To perform the foregoing subpixel-processing, it is preferable to heighten the contrast between the background 70a and the optotype 72 or 73 as much as possible. And further, to reduce the influence of the color of the boundary of the optotype 72 or 73 and the background, it is preferable that the color of the optotype 72 or 73 be chosen between white color and the black color. On the other hand, it is preferable that the color of the background 70a be chosen between white color and black color such that the background 70 differs from the optotype 72 or 73 in color.

In the above-example, the position of the outline of the optotype 72 or 73 can be set at 24 ($8 \times 3$) positions at one pixel (the pixel 82) because the brightness of one subpixel is equally divided into 8 steps. Therefore, in a case where a display including about-0.19-mm-wide pixels is used as the display 30, the position of the outline of the optotype 72 or 73 can be adjusted in steps of about 0.008 millimeters.

Operation of the optotype representing apparatus 100 having the foregoing configuration at the time of a trirod vision test will be described below. To begin with, an examiner performs settings on the apparatus 100 before the test. At that time, the test distance (the test position of an examinee) DE and the fusion distance FD are determined. Then data on test distance (the test position of the examinee) DE and the fusion distance FD is input to the apparatus 100 by using the keys 64 and 66 of the remote control 60 or the function key. Also, data on a pupillary distance PD of the examinee is input to the apparatus 100 by using the key 65 of the remote control 60 or the function key 12. The pupillary distance PD is measured in advance by using a pupillostatometer or the like. The control unit 40 stores the test distance DE, the fusion distance FD, and the pupillary distance PD in the memory 41 based on a command signal from the remote control 60 or the function key 12.

Then the examiner places the examinee wearing the polarizing spectacles 90 or being in a state in which the phoropter 200 is placed in front of the left and right eyes at the predetermined test position. Thereafter, the trirod vision-testing optotype 70 is presented on the optotype presenting part 30 (the display 31) through the use of the key 61 by the examiner. The control unit 40 calls up (reads) the test distance DE, the fusion distance FD, and the pupillary distance PD stored in the memory 41 based on a command signal from the remote control 60, following which the control unit 40 determines the inter-optotype spacing W2 for the optotype 72 and the inter-optotype spacing W3 for the optotype 73 based on the test distance DE and the pupillary distance PD: the inter-optotype spacing W2 for the optotype 72 and the inter-optotype spacing W3 for the optotype 73 takes on a value at which the fusion distance for (the flotage amount or the sinkage amount of) the optotype 70 corresponds with the fusion distance FD set (input) in advance. Then the control unit 40 forms an optotype 70 (optotypes 71, 72, and 73) based on the inter-optotype spacings W2 and W3, and then performs subpixel-processing at the display 31 to display the optotype 70. Thereafter, the left eye EL of the examinee perceives the images of the left-eye optotypes 72L and 73L in the optotype 70via the optical region 32a and the polarizing filter 91L or 201L. Also, the right eye ER of the examiner perceives the images of the right-eye optotypes 72R and 73R via the optical region 32b and the polarizing filter 91R or 201R. Likewise, the left and right eyes EL and ER of the examinee perceive the image of the optotype 71. Here, the optotype 71 perceived by the examiner is positioned on the screen of the display 31 (i.e., positioned at a distance of 2.5 meters from the examinee). The examinee sees (perceives) the optotype 72 floating from the optotype 71. On the other hand, the examinee sees (perceives) the optotype 73 sinking from the optotype 71.

The examiner determines the examiner's deep vision as follows, and then determines whether the trirod vision test result is good or bad. That is, the examiner asks the examinee how the examinee sees the three optotype 71, 72, and 73. In the case where the examinee perceives the optotype 72 as if the optotype 72 is in front of (is on the near side of) the optotype 71 or perceives the optotype 73 as if the optotype 73 is behind (on the far side of) the optotype 71, it is determined that the examinee has a good vision.

Through the optotype presenting apparatus 100 according to this embodiment, trirod vision tests can be conducted using such a simple method as described above. Moreover, an optotype is seen floating or sinking by a fixed fusing distance regardless of the pupillary distances of an examinee. Hence vision tests can be performed with high accuracy.

In the above description, at the optotype presenting apparatus, three kinds of optotypes are used as a trirod vision-testing optotype (image); however, such a trirod testing optotype is not limited to the above three kinds of optotypes. That is, any of such an optotype used as a test reference, such an optotype seen (perceived) floating with respect to the reference optotype by a predetermined amount, such an optotype seen (perceived) sinking with respect to the reference optotype by a predetermined amount may be included.

As described above, at the optotype presenting apparatus, data on an optotype is stored in the memory as vector data (a vector image), and the control unit determines an inter-optotype spacing based on the setting (the input) of a pupillary distance to display the optotype on the display. However, processing performed at the optotype presenting apparatus is not limited to the above processing. At the optotype presenting apparatus, a plurality of optotype image corresponding to different pupillary distances can be stored in the memory 41, and then the control unit 40 can read the suitable optotype image data from the memory 41 based on the input of the pupillary distance data to display the optotype image on the display 31. Incidentally, data on a test distance and a fusion distance can also be input as in the case of the pupillary distances.

The configuration and operation of the optotype presenting apparatus has been described with regard to the case where the test distance corresponds with the installation distance; however, the use of the optotype presenting apparatus is not limited to only such a case. A fusion distance for an optotype can be set at any value provided that the optotype is seen floating or sinking from a position used as a test reference. Therefore, for example, the installation distance can be set to 5.0 meters provided that examinees see the reference optotype 71 floating from the installation distance by 2.5 meters, see the optotype 72 floating from the optotype 71 by the fusion distance, and see the optotype 73 sinking from the optotype 71 by the fusion distance.

The optotype presenting apparatus, as described earlier, includes the polarization optical member, the polarizing filter, and so on to separate an optotype to be presented to the left and right eyes of an examinee. However, a unit that separates an optotype by using circular polarization, for example, may be used to separate an optotype. Furthermore, in the foregoing description, the polarization optical member placed on the front of the display and the polarizing spectacles or the phoropter set in front of the eyes of the examinee are used to separate an optotype to be presented to the left and right eyes of the examinee. However, examples of a unit for optotype separation are not limited to these components; that is, a unit that separates an optotype by using color, a unit that separates an optotype by using a liquid crystal shutter, or the like may be used. For example, the color of an optotype to be presented to examinees' left eyes can be set at red, and the color of an optotype to be presented to examinees' right eyes can be set at green. In that case, the examinee is made to put on red-green spectacles (spectacles set with a red filter for left eyes and set with a green filter for right eyes) to take a vision test, or the phoropter can be set with a red filter and a green filter as in the case of the spectacles. Incidentally, the colors of the filters are not limited to red and green. Furthermore, examinees can be made to put on spectacles having a liquid crystal shutter function. Such spectacles can make light to be emitted to examinee's left and right eyes pass through the spectacles themselves and can intercept the light alternately at predetermined time intervals. By using the spectacles, a left-eye optotype and a right-eye optotype can be alternately represented (displayed) on the display at predetermined time intervals.

In the optotype presenting apparatus according to this embodiment, as described earlier, the pixels of the display 31 are subjected to subpixel-processing to adjust the position of the outline of an optotype; however, a method for adjusting the position of the outline of an optotype is not limited to the above method. For example, the pixels positioned at the boundary portion of an optotype can each be subjected to subpixel-processing. Specifically, the brightness of pixels is changed on a gray scale. In other words, the brightness levels of the subpixels of pixels may be changed to the same level. Therefore a color display is not necessarily used; a monochrome display can be used.

As described earlier, the optotype presenting apparatus presents an optotype to an examinee to conduct simple trirod vision tests; however, the optotype presenting apparatus can be used to test the examinee's binocular visual function such as stereoscopic visual function.

As optotype presenting apparatuses other than the optotype presenting apparatus according to this embodiment, optotype presenting apparatuses according to first to ninth embodiments of the present invention can also be implemented.

A first optotype presenting apparatus that presents optotypes for testing an examinee's binocular visual function, the apparatus comprising:

an optotype presenting part to display an optotype at a predetermined presenting region;
a pupillary distance input part that inputs a pupillary distance between an examinee's left and right eyes; and
a control unit that makes the optotype presenting part display a left-eye optotype and a right-eye optotype for a stereoscopic vision test,
wherein the control unit determines a lateral inter-optotype spacing between the left-eye optotype and the right-eye optotype based on the input pupillary distance so that a fusion optotype seen with the examinee's both eyes is perceived as an optotype that is seen floating or sinking by a fusion distance between a reference position and a predetermined fusion position, and makes the optotype presenting part display the left-eye optotype and the right-eye optotype based on the determined inter-optotype spacing.

A second optotype presenting apparatus is configured according to the configuration of the first optotype presenting apparatus, wherein the control unit divides a product of the pupillary distance and the fusion distance by a distance between the examinee and the fusion position to determine the inter-optotype spacing.

A third optotype presenting apparatus is configured according to the configuration of the first optotype presenting apparatus, wherein the control unit determines brightness of pixels corresponding to longitudinal portions of a perimeter of at least one of the right-eye optotype and the left-eye optotype to make the examinee perceive the inter-optotype spacing as a distance shorter than a value that is an integral multiple of a width of each pixel included in the optotype presenting part, and changes the brightness of the pixels based on the inter-optotype spacing.

A fourth optotype presenting apparatus is configured according to the configuration of the first optotype presenting apparatus, wherein the optotype presenting part includes a color display on which a plurality of subpixels different in color of individual pixels are arranged laterally; and

in order to make the examinee perceive the inter-optotype spacing as a distance shorter than a value that is an integral multiple of a width of each pixel, the control unit changes brightness of the subpixels of the pixels corresponding to longitudinal portions of a perimeter of at least one of the right-eye optotype and the left-eye optotype from a side of the optotype to a side of a background, based on the determined inter-optotype spacing.

A fifth optotype presenting apparatus is configured according to the configuration of the fourth optotype presenting apparatus, wherein the control unit makes the optotype presenting part display the optotype in black or white color.

A sixth optotype presenting apparatus is configured according to the configuration of the fifth optotype presenting apparatus wherein the control unit makes the optotype presenting part display a background for the optotype in color opposite to the color of the optotype.

A seventh optotype presenting apparatus is configured according to the configuration of the first optotype presenting apparatus, the apparatus further comprising a fusion distance input part that inputs data on the fusion distance from the reference position to the predetermined fusion position.

An eight optotype presenting apparatus is configured according to the configuration of the first optotype presenting apparatus wherein the left-eye optotype and the right-eye optotype are identical in shape, size, and color.

A ninth optotype presenting apparatus is an optotype presenting apparatus that presents optotypes for testing an examinee's binocular visual function, the apparatus comprising:

a display for displaying an optotype;
a pupillary distance input part for inputting a pupillary distance between an examinee's left and right eyes; and
a control unit for allowing the display to display a left-eye optotype and a right-eye optotype for a stereoscopic vision test
wherein the control unit determines a lateral inter-optotype spacing between the left-eye optotype and the right-eye optotype based on the input pupillary distance so that a fusion optotype seen with the examinee's both eyes is perceived as an optotype that is seen floating or sinking by a fusion distance between a reference position and a predetermined fusion position, and allows the display to display the left-eye optotype and the right-eye optotype based on the determined inter-optotype spacing.

According to a ninth embodiment of the invention, there is provided an optotype presenting apparatus that presents an optotype for testing an examinee's binocular visual function. The optotype presenting apparatus includes a display to display the optotype, a pupillary distance input part to input a pupillary distance between the examinee's left and right eyes, and control unit that makes the display display a left-eye optotype and a right-eye optotype for a stereoscopic vision test, that determines a lateral inter-optotype spacing between the left-eye optotype and the right-eye optotype based on the input pupillary distance so that a fusion optotype seen with the examinee's both eyes is perceived floating or sinking by a fusion distance between a reference position and a predetermined fusion position, and that makes the display display the left-eye optotype and the right-eye optotype based on the determined inter-optotype spacing.

In these optotype presenting apparatuses, by adjusting each apparatus so that the fusion position of optotypes that produce a parallax is set optimally based on a pupillary distance of an examinee, a vision test can be conducted with higher accuracy.

## Claims

1. An optotype presenting apparatus that presents optotypes for testing an examinee's binocular visual function, the apparatus comprising:

   an optotype presenting part to display an optotype at a predetermined presenting region;
   a pupillary distance input part that inputs a pupillary distance between an examinee's left and right eyes; and
   a control unit that makes the optotype presenting part display a left-eye optotype and a right-eye optotype for a stereoscopic vision test,
   wherein the control unit determines a lateral inter-optotype spacing between the left-eye optotype and the right-eye optotype based on the input pupillary distance so that a fusion optotype seen with the examinee's both eyes is perceived as an optotype that is seen floating or sinking by a fusion distance between a reference position and a predetermined fusion position, and makes the optotype presenting part display the left-eye optotype and the right-eye optotype based on the determined inter-optotype spacing.

2. The optotype presenting apparatus according to claim 1, wherein
   the control unit divides a product of the pupillary distance and the fusion distance by a distance between the examinee and the fusion position to determine the inter-optotype spacing.

3. The optotype presenting apparatus according to claim 1 or 2, wherein
   the control unit determines brightness of pixels corresponding to longitudinal portions of a perimeter of at least one of the right-eye optotype and the left-eye optotype to make the examinee perceive the inter-optotype spacing as a distance shorter than a value that is an integral multiple of a width of each pixel included in the optotype presenting part, and changes the brightness of the pixels based on the inter-optotype spacing.

4. The optotype presenting apparatus according to any one of claims 1 to 3, wherein
   the optotype presenting part includes a color display on which a plurality of subpixels different in color of individual pixels are arranged laterally; and
   in order to make the examinee perceive the inter-optotype spacing as a distance shorter than a value that is an integral multiple of a width of each pixel, the control unit changes brightness of the subpixels of the pixels corresponding to longitudinal portions of a perimeter of at least one of the right-eye optotype and the left-eye optotype from a side of the optotype to a side of a background, based on the determined inter-optotype spacing.

5. The optotype presenting apparatus according to claim 4, wherein
   the control unit makes the optotype presenting part display the optotype in black or white color.

6. The optotype presenting apparatus according to claim 5, wherein
   the control unit makes the optotype presenting part display a background for the optotype in color opposite to the color of the optotype.

7. The optotype presenting apparatus according to any one of claims 1 to 6, the apparatus further comprising:

   a fusion distance input part that inputs data on the fusion distance from the reference position to the predetermined fusion position.

8. The optotype presenting apparatus according to any one of claims 1 to 6, wherein
   the left-eye optotype and the right-eye optotype are identical in shape, size, and color.

# FIG. 1

# FIG. 2

# FIG. 3

# FIG. 4

# FIG. 5

# FIG. 6

Europäisches
Patentamt

European
Patent Office

Office européen
des brevets

# EUROPEAN SEARCH REPORT

**Application Number**

EP 12 16 2255

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 825 456 A (TABATA SEIICHIRO [JP] ET AL) 20 October 1998 (1998-10-20) | 1,2 | INV. A61B3/032 |
| A | * column 4, line 62 - column 10, line 52 * ----- | 3-8 | A61B3/08 A61B3/11 |
| X | EP 0 751 689 A2 (MATSUSHITA ELECTRIC IND CO LTD [JP]) 2 January 1997 (1997-01-02) * column 20, line 53 - column 23, line 53 * <br><br> * column 28, line 43 - line 53 * ----- | 1 | H04N13/00 |
| A | WO 2010/125908 A1 (TOPCON CORP [JP]; SAKURADA TOMOHIRO [JP]; AKIYAMA HISANORI [JP]) 4 November 2010 (2010-11-04) * the whole document * ----- | 1-8 | |
| A | US 5 034 809 A (KATOH HIDEAKI [JP]) 23 July 1991 (1991-07-23) * column 3, line 15 - column 4, line 45 * ----- | 1 | |
| A | US 5 530 492 A (RON SAMUEL [IL]) 25 June 1996 (1996-06-25) * column 8, line 23 - line 39 * ----- | 1 | **TECHNICAL FIELDS SEARCHED (IPC)** <br><br> A61B <br> H04N |

The present search report has been drawn up for all claims

| Place of search | Date of completion of the search | Examiner |
|---|---|---|
| Munich | 4 July 2012 | Alvazzi Delfrate, S |

**ANNEX TO THE EUROPEAN SEARCH REPORT**
**ON EUROPEAN PATENT APPLICATION NO.**                        EP 12 16 2255

This annex lists the patent family members relating to the patent documents cited in the above-mentioned European search report.
The members are as contained in the European Patent Office EDP file on
The European Patent Office is in no way liable for these particulars which are merely given for the purpose of information.

04-07-2012

| Patent document cited in search report | | Publication date | Patent family member(s) | | Publication date |
|---|---|---|---|---|---|
| US 5825456 | A | 20-10-1998 | JP | 8322004 A | 03-12-1996 |
| | | | US | 5825456 A | 20-10-1998 |
| EP 0751689 | A2 | 02-01-1997 | DE | 69619308 D1 | 28-03-2002 |
| | | | DE | 69619308 T2 | 11-07-2002 |
| | | | DE | 69631496 D1 | 11-03-2004 |
| | | | DE | 69631496 T2 | 01-07-2004 |
| | | | DE | 69632755 D1 | 22-07-2004 |
| | | | DE | 69632755 T2 | 04-11-2004 |
| | | | EP | 0751689 A2 | 02-01-1997 |
| | | | EP | 1168852 A1 | 02-01-2002 |
| | | | EP | 1328129 A1 | 16-07-2003 |
| | | | US | 6005607 A | 21-12-1999 |
| | | | US | 6175379 B1 | 16-01-2001 |
| | | | US | 6268880 B1 | 31-07-2001 |
| | | | US | 2001033327 A1 | 25-10-2001 |
| | | | US | 2002024592 A1 | 28-02-2002 |
| WO 2010125908 | A1 | 04-11-2010 | JP | 2010259495 A | 18-11-2010 |
| | | | WO | 2010125908 A1 | 04-11-2010 |
| US 5034809 | A | 23-07-1991 | JP | 2281891 A | 19-11-1990 |
| | | | JP | 3129719 B2 | 31-01-2001 |
| | | | US | 5034809 A | 23-07-1991 |
| US 5530492 | A | 25-06-1996 | NONE | | |

For more details about this annex : see Official Journal of the European Patent Office, No. 12/82

**EP 2 505 126 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011081288 A
- JP 2009207569 A
- EP 2095760 A